# EUROPÄISCHE PATENTANMELDUNG

(11) **EP 0 646 637 A1**
(43) Veröffentlichungstag der Anmeldung: **05.04.1995**
(21) Anmeldenummer: 94115106.0
(22) Anmeldetag: 26.09.1994
(51) Int. Cl.: C10M 105/34, C10M 105/38, C09K 5/04, C07C 45/49, C07C 51/145, C07C 53/126, C07C 53/128

(54) **Gemische isomerer Pentansäuren, ihre Ester und deren Verwendung als Schmiermittel.**

(30) Priorität: 30.09.1993 DE 4333323
(71) Anmelder: HOECHST AKTIENGESELLSCHAFT, D-65926 Frankfurt am Main (DE)
(72) Erfinder: Bahrmann, Helmut, Dr., D-46499 Hamminkeln (DE); Greb, Wolfgang, Dr., D-46535 Dinslaken (DE); Dämbkes, Georg, Dr., D-46537 Dinslaken (DE); Heymanns, Peter, Dr., D-45147 Essen (DE); Kalbfell, Heinz, D-46514 Schermbeck (DE); Lappe, Peter, Dr., D-46539 Dinslaken (DE); Springer, Helmut, D-46145 Oberhausen (DE); von Mülmann, Eberhard, D-46147 Oberhausen (DE); Weber, Jürgen, Dr., D-46147 Oberhausen (DE); Wiebus, Ernst, D-46147 Oberhausen (DE); Frohning, Carl Dieter, Dr., D-46485 Wesel (DE); Kappesser, Harald, D-46149 Oberhausen (DE)

(57) **Zusammenfassung**

Gemische isomerer Pentansäuren werden durch Hydroformylierung von Buten-1/Buten-2 Gemischen zu Aldehydgemischen und Oxidation der abgetrennten Aldehydgemische hergestellt. Die Hydroformylierung erfolgt in Gegenwart von Rhodium-Katalysatoren, die in Wasser gelöst sind. Das Gemisch isomerer C₅-Monocarbonsäuren ergibt, mit zwei- oder mehrwertigen Alkoholen verestert, ein Gemisch isomerer Ester, die als Schmiermittel für Kältemittelverdichter, die mit chlorfreien, teilfluorierten Kohlenwasserstoffen als Kältemittel betrieben werden, Anwendung finden.

## Beschreibung

Die Erfindung betrifft Gemische isomerer, aliphatischer C₅-Monocarbonsäuren, ein Verfahren zu ihrer Herstellung, die aus diesen Säuren erhaltenen Ester und deren Verwendung als Schmiermittel für Kältemittelverdichter, die mit chlorfreien, teilfluorierten Kohlenwasserstoffen als Kältemittel betrieben werden.

Zur Kälteerzeugung im industriellen, gewerblichen wie auch im privaten Bereich werden in großem Umfang Kältemittelverdichter eingesetzt. Diese Vorrichtungen arbeiten mit mechanischen Verdichtern, die das Kältemittel komprimieren, im Verflüssiger durch Kühlen mit Luft, Wasser oder einem anderen Medium verflüssigen und im Verdampfer unter Wärmeaufnahme aus dem zu kühlenden Medium verdampfen. Als Kältemittel werden vorwiegend Ammoniak für Großanlagen und Fluorchlorkohlenwasserstoffe wie Dichlordifluormethan, Chlortrifluormethan für Großanlagen, gewerbliche Kälteanlagen und für Haushaltsgeräte eingesetzt.

Zur Schmierung von Kältemittelverdichtern verwendet man hochraffinierte, weißölähnliche, im allgemeinen naphthenbasische Mineralöle. Als vollsynthetische Öle für Kältemittelverdichter gelangen Alkylaromaten und daneben auch Poly-alpha-olefine zum Einsatz.

Aufgabe der Schmieröle ist es, die beweglichen Verdichterteile zu schmieren, die Wärme von den heißen Verdichterteilen abzuführen und den Kompressionsraum sowie die Ventile abzudichten. Diese Aufgaben bestimmen auch die Eigenschaften, denen die Schmieröle genügen müssen. Sie müssen thermischen Belastungen gewachsen sein und auch bei den Temperaturen des Verdampfers fließfähig bleiben. Überdies ist zu berücksichtigen, daß die Schmieröle aus dem Kompressionsraum in den Kältemittelkreislauf ausgetragen werden und durch nachgeschaltete Ölabscheider nicht vollständig entfernt werden können. Sie müssen daher mit dem Kältemittel in weiten Temperatur- und Konzentrationsbereichen mischbar sein, so daß die Rückführung von Schmieröl, das in den Kältemittelkreislauf gelangt ist, in den Verdichter sichergestellt wird.

Fluorchlorkohlenwasserstoffe stehen seit einiger Zeit im Verdacht, die Ozonschicht der Erdatmosphäre zu schädigen. Daher ist man bestrebt, ihre Verwendung auf solche Fälle zu beschränken, in denen sie nicht ausgetauscht werden können. Im übrigen versucht man, sie durch gleichwirkende, jedoch unschädliche Stoffe zu ersetzen. Auf dem Gebiet der Kältemittel für Kälteanlagen werden zukünftig chlorfreie, teilfluorierte Kohlenwasserstoffe wie 1,1,1,2-Tetrafluorethan, 1,1,1,2,3,3,3-Heptafluorpropan, Pentafluorethan, 1,1,1,3,3,3-Hexafluorpropan und Trifluormethan eingesetzt werden. Diese Stoffe zeichnen sich durch hohe thermische Stabilität und entsprechende thermodynamische Eigenschaften in den Temperaturbereichen aus, die beim Betrieb von Kältemittelverdichtern auftreten.

Die chlorfreien aliphatischen Fluorkohlenwasserstoffe sind mit den bisher verwendeten Schmiermitteln für Kälteanlagen nur in geringem Umfang mischbar. In weiten Konzentrationsbereichen bilden die beiden Substanzklassen Zweiphasengemische, so daß die Rückführung des aus dem Verdichter ausgetragenen Schmiermittels vor allem bei tiefen Temperaturen erheblich behindert wird. Es mußten daher neue, mit den chlorfreien Kältemitteln verträgliche Schmiermittel aufgefunden werden. Unter ihnen haben sich aus Monocarbonsäuren und zwei- oder mehrwertigen Alkoholen erhaltene Ester ausgezeichnet bewährt (vgl. DE 40 06 827 A1). Neuere Untersuchungen zielen darauf ab, Schmiermittel aus leichtzugänglichen Rohstoffen und, im Hinblick auf unterschiedliche Kältemittel, mit möglichst vielfältigen Einsatzmöglichkeiten zu entwickeln.

Es bestand somit die Aufgabe preiswerte Ausgangsstoffe bereitzustellen, die sich auf konventionellem Wege in Schmiermittel überführen lassen, die mit unterschiedlichen Kältemitteln verwendet werden können.

Überraschenderweise hat sich gezeigt, daß ausgewählte Mischungen isomerer Carbonsäuren zusammen mit zwei- oder mehrwertigen aliphatischen Alkoholen Esterschmiermittel ergeben, die hohe Wirtschaftlichkeit mit mannigfacher Verwendbarkeit vereinen.

Die Erfindung besteht in Gemischen isomerer Pentansäuren erhalten durch Hydroformylierung von Buten-1/Buten-2-Gemischen in einem heterogenen Reaktionssystem unter Verwendung wasserlösliche Phosphine in komplexer Bindung enthaltender Rhodiumverbindungen als Katalysatoren bei Temperaturen von 70 bis 150°C und Drücken von 0,4 bis 30 MPa zu Aldehydgemischen, Abtrennung der erhaltenen Aldehydgemische aus dem Hydroformylierungsprodukt und Oxidation zu einem Gemisch isomerer Pentansäuren.

Die zur Herstellung der erfindungsgemäßen Gemische isomerer C₅-Monocarbonsäuren eingesetzten Buten-1 und Buten-2 enthaltenden Gemische fallen als Raffinerienebenprodukte bei der Herstellung von Automobiltreibstoffen und bei der Herstellung von Ethylen durch thermische Spaltung höherer Kohlenwasserstoffe zwangsweise in erheblichen Mengen an. Man gewinnt sie aus den C₄-Crackschnitten des Pyrolyseproduktes durch Extraktion von Butadien-1,3 mit einem selektiven Lösungsmittel und anschließende Abtrennung des Isobutens vorzugsweise durch Umwandlung in Methyl-tert.-butylether. Das von Butadien befreite Pyrolyseprodukt wird als Raffinat I bezeichnet. Ist darüberhinaus auch noch das Isobuten abgetrennt, spricht man von Raffinat II. Statt das Butadien zu extrahieren, kann es auch im C₄-Crackschnitt partiell zu Butenen hydriert werden. Nach Abtrennung des i-Butens erhält man ein Buten-1/Buten-2-Gemisch, das besonders geeignet zur Weiterverarbeitung auf C₁₀-Alkohole ist. Schließlich geht man in letzter Zeit auch dazu über, das abgetrennte Butadien zu Butan zu hydrieren und in die Spaltung zurückzuführen, um die Ethylen- und Propylenausbeute zu erhöhen.

Erfindungsgemäß werden Buten-1 und Buten-2 enthaltende Gemische z.B. in Form des Raffinats II, aber auch anderer Herkunft und Zusammensetzung hydroformyliert. Hierbei wird bevorzugt Buten-1 umgesetzt zu einem Gemisch, das vorwiegend aus n-Valeraldehyd und, in untergeordneter Menge, aus i-Valeraldehyd besteht. Die Reaktion verläuft unter Bedingungen, die eine Isomerisierung des Buten-1 zu Buten-2 weitgehend ausschließen. Nicht umgesetzte Olefine, vorwiegend Buten-2, können zur Vervollständigung der Reaktion einer zweiten Hydroformylierungsstufe zugeführt werden.

Die Hydroformylierung führt man erfindungsgemäß als heterogene Reaktion in einem Zweiphasensystem durch, eine Umsetzung, die z.B. in der DE-PS 26 27 354 beschrieben ist. Dieser Prozeß ist charakterisiert durch das Vorliegen einer organischen Phase, die die Ausgangsolefine und das Reaktionsprodukt enthält und einer wäßrigen Phase, in der der Katalysator gelöst ist. Als Katalysatoren werden wasserlösliche Rhodiumkomplexverbindungen eingesetzt, die wasserlösliche Phosphine als Liganden enthalten. Zu den Phosphinen zählen insbesondere Triarylphosphine, Trialkylphosphine und arylierte bzw. alkylierte Diphosphine, deren organische Reste durch Sulfonsäuregruppen oder Carboxylgruppen substituiert sind. Ihre Herstellung ist z.B. aus DE-PS 26 27 354 und DD-PS 259 194 bekannt. Die Reaktion der Butene erfolgt bei Temperaturen von 70 bis 150°C, vorzugsweise 100 bis 130°C und unter Drücken im Bereich von 0,4 bis 30, insbesondere 1 bis 10 MPa mit Wassergas, das Kohlenmonoxid und Wasserstoff im Volumenverhältnis 1 : 10 bis 10 : 1 enthält. Die Rhodiumkonzentration beträgt 20 bis 1000 Gew.-ppm, vorzugsweise 50 bis 500 Gew.-ppm, bezogen auf die wäßrige Katalysatorlösung. Je mol Rhodium setzt man 4 bis 100 mol wasserlösliches Phosphin ein. Das Volumenverhältnis von wäßriger zu organischer Phase beträgt 0,1 bis 10 : 1.

Der Butenumsatz je Zeiteinheit wird deutlich erhöht, wenn man der wäßrigen Katalysatorlösung ein Phasentransferreagenz (Lösungsvermittler) zusetzt. Es verändert die physikalischen Eigenschaften der Grenzflächen zwischen den beiden flüssigen Phasen und erleichtert den Übergang des organischen Reaktanten in die wäßrige Katalysatorphase.

Als Lösungsvermittler sind Verbindungen bekannt, deren hydrophile Gruppen ionisch (anionisch oder kationisch) oder nichtionisch sind. Zu den anionenaktiven Verbindungen gehören Natrium-, Kalium- oder Ammoniumsalze von Carbonsäuren mit 8 bis 20 Kohlenstoffatomen, insbesondere von gesättigten Fettsäuren mit 12 bis 18 Kohlenstoffatomen, ferner Alkylsulfate, Alkylbenzolsulfonate und Alkylbenzolphosphate. Beispiele für kationische Lösungsvermittler sind Tetraalkylammonium- und N-Alkylpyridiniumsalze. Die nichtionischen Phasentransferreagentien können in wäßriger Lösung nicht in Ionen dissoziieren. Zu ihnen zählen Alkylpolyethylenglykole, Alkylphenylpolyethylenglykole, Fettsäurealkylolamide und Trialkylaminoxide. Schließlich finden auch Ampholyte wie Aminocarbonsäuren, Betaine und Sulfobetaine als Lösungsvermittler Anwendung.

Bewährt haben sich insbesondere kationische Lösungsvermittler der allgemeinen Formel [A-N(R¹R²R³)]⁺E⁻, in der A für einen geradkettigen oder verzweigten Alkylrest mit 6 bis 25 Kohlenstoffatomen steht, R¹, R², R³ gleich oder verschieden sind und geradkettige oder verzweigte Alkylreste mit 1 bis 5 Kohlenstoffatomen bedeuten und E für ein Anion, insbesondere für Sulfat, Tetrafluorborat, Acetat, Methosulfat, Benzolsulfonat, Alkylbenzolsulfonat, Toluolsulfonat, Lactat oder Citrat steht.

Nach Beendigung der Hydroformylierung wird das Aldehydgemisch vom Katalysator, von den nichtumgesetzten Reaktionsteilnehmern und den übrigen Reaktionsprodukten durch einfache Phasentrennung abgeschieden.

In der darauffolgenden Reaktionsstufe werden die Aldehydgemische zu einem Gemisch isomerer Pentansäuren oxydiert. Die Umsetzung erfolgt in bekannter Weise durch Behandlung der Aldehyde mit Sauerstoff in reiner Form oder in Mischung mit inerten Gasen in Abwesenheit oder, vorzugsweise, in Gegenwart von Katalysatoren. Als Katalysatoren kommen vorwiegend Salze der Übergangsmetalle in Betracht, insbesondere Salze des Kobalts und des Mangans sowie des Chroms, Eisens, Kupfers, Nickels, Silbers und Vanadiums. Zur Vermeidung von Abbau- und Nebenreaktionen wird die Oxidation bei möglichst niedrigen Temperaturen vorgenommen. Überdies läßt sich die Selektivität der Reaktion durch Zusatz von Alkalisalzen schwacher Carbonsäuren z.B. Kaliumpentanoat verbessern. Zur Reingiung wird das Säuregemisch destilliert. Es eignet sich vorzüglich als Säurekomponente in Esterschmiermitteln.

Als Alkoholkomponente enthalten die Ester zwei- oder mehrwertige Alkohole. Beispiele für zweiwertige Alkohole sind Ethylenglykol, Diethylenglykol, Triethylenglykol, Propandiol-1,2, Dipropylenglykol, Tripropylenglykol, Propandiol-1,3, Butandiol-1,3, Butandiol-1,4, Neopentylglykol, Hexandiol-1,6, 3(4),8(9)-Bis(hydroxymethyl)-tricyclo-[5.2.1.0.^{2.6}]decan und 1,4-Dimethylolcyclohexan. Unter den mehrwertigen Alkoholen haben insbesondere die drei- und vierwertigen Bedeutung, für die als Beispiele Trimethylolpropan (2-Ethyl-2-hydroxymethyl-1,3-propandiol), Glycerin und Pentaerythrit (2,2-Bis(hydroxymethyl)-1,3-propandiol) genannt seien. Zur Herstellung der zwei- und mehrwertigen Alkohole geht man von individuellen Verfahren aus, die dem Fachmann bekannt sind.

Besondere Bedeutung haben Ester des Neopentylglykols mit Monocarbonsäuren.

Die Ester werden in bekannter Weise aus den vorgenannten Säuren und Alkoholen in Gegenwart von sauren Katalysatoren hergestellt. Als Katalysatoren eignen sich Mineralsäuren wie Schwefelsäure, Phosphorsäure, sowie deren saure Salze, ferner Trialkyl- oder Triarylphosphate und p-Toluolsulfonsäure. Um eine möglichst vollständige Umsetzung zu erzielen, empfiehlt es sich, einen der Reaktionspartner im Überschuß anzuwenden und/oder das Reaktionswasser destillativ, gegebenenfalls unter Zusatz eines Azeotropbildners wie Benzol, Toluol oder Cyclohexan, abzutrennen.

Die erfindungsgemäß verwendeten Schmiermittel besitzen gute Mischbarkeit mit den als Kältemitteln eingesetzten chlorfreien, teilfluorierten Kohlenwassertoffen auch noch bei -40°C, also in den Temperaturbereichen, die in Kompressionskälteanlagen auftreten können. Ihre Viskosität liegt bei 40°C zwischen etwa 10 und 100 mm²/sec und entspricht damit den Anforderungen, die an das Schmiermittel für das vorbeschriebene Einsatzgebiet gestellt werden. Sie weisen darüber hinaus unter Ausschluß von Luftsauerstoff und Feuchtigkeit, also unter Bedingungen, die in einem Kältemittelkreislauf erfüllt sein müssen, ausgezeichnete thermische Stabilität auf. Die Ester sind nicht hygroskopisch. Sie können daher ohne großen Aufwand getrocknet werden. Restfeuchten, die nach der deutschen Norm DIN 51 503 35 ppm nicht überschreiten dürfen, lassen sich ohne weiteres erreichen.

Die als Schmiermittel verwendeten isomeren Ester können als solche oder auch in Mischung mit zwei oder mehr Estern anderer chemischer Zusammensetzung eingesetzt werden.

### Beispiel 1

Ein Gemisch isomerer Pentansäuren (4,2 mol) wird mit Pentaerythrit (1 mol) in Gegenwart von p-Toluolsulfonsäure (0,01 mol) als Katalysator und Cyclohexan zur Entfernung des Reaktionswassers als Azeotrop bei 140 °C über einen Zeitraum von 4 h umgesetzt.

Das Reaktionsprodukt wird mit Natronlauge (5 Gew.-% NaOH) neutralisiert. Man trennt wässrige und organische Phase voneinander, wäscht die organische Phase mit Wasser und gibt nochmals Natronlauge (5 Gew.-% NaOH) bis zur Einstellung eines pH-Wertes von 9 bis 10 hinzu. Die organische Phase wird wiederum abgetrennt, mit Wasser gewaschen und schließlich destilliert.

Kriterien, die die Eignung des Estergemisches als Schmiermittel beurteilen lassen, sind seine Viskosität und seine Mischbarkeit mit 1,1,1,2-Tetrafluorethan (R 134a) als Prototyp eines Kältemittels.

Die Messung der Viskosität des Estergemisches erfolgt in einem Ubbelohde-Viskosimeter bei 40 °C.

Zur Prüfung der Mischbarkeit des Estergemisches mit dem Kältemittel gibt man eine definierte Menge Ester (0,2 bis 3,0 g) in ein Glasröhrchen von etwa 10 ml Inhalt. Nach Eintauchen in flüssigen Stickstoff kondensiert man je nach einzustellender Konzentration zwischen 6 und 3 g des Kältemittels dazu. Darauf evakuiert man das Glasröhrchen, verschließt es durch Abschmelzen und durchschreitet mit der Mischung bestimmter Zusammensetzung einen Temperaturbereich von -40 °C bis +80°C. Bei Ausbildung zweier Phasen bzw. bei beginnender Trübung kann der Entmischungspunkt, d.h. ein Punkt auf der Grenzkurve des Mischungsdiagramms bestimmt werden. Die Gesamtheit der gefundenen Entmischungspunkte ergeben dann die Grenzkurve der Mischungslücke.

Ergebnis: das Estergemisch hat bei 40°C eine kinematische Viskosität von 16,9 mm²/s und zeigt bis -30 °C keine Mischungslücken mit R 134a.

## Patentansprüche

1. Gemische isomerer Pentansäuren, erhalten durch Hydroformylierung von Buten-1/Buten-2 Gemischen in einem heterogenen Reaktionssystem unter Verwendung wasserlösliche Phosphine in komplexer Bindung enthaltender Rhodiumverbindungen als Katalysatoren bei Temperaturen von 70 bis 150°C und Drücken von 0,4 bis 30 MPa zu Aldehydgemischen, Abtrennung der erhaltenen Aldehydgemische aus dem Hydroformylierungsprodukt und Oxidation zu einem Gemisch isomerer Pentansäuren.

2. Gemische isomerer Pentansäuren nach Anspruch 1, dadurch gekennzeichnet, daß die Umsetzung in der ersten Hydroformylierungsstufe bei 100 bis 130°C und unter Drücken von 1 bis 10 MPa erfolgt.

3. Gemische isomerer Pentansäuren nach Anspruch 1 oder 2, dadurch gekennzeichnet, daß die Umsetzung in der Hydroformylierungsstufe bei einer Rhodiumkonzentration von 20 bis 1000 Gew.-ppm, vorzugsweise 50 bis 500 Gew.-ppm, bezogen auf die wäßrige Katalysatorlösung, erfolgt.

4. Gemische isomerer Pentansäuren nach einem oder mehreren der Ansprüche 1 bis 3, dadurch gekennzeichnet, daß in der Hydroformylierungsstufe je mol Rhodium 4 bis 100 mol wasserlösliches Phosphin eingesetzt werden.

5. Gemische isomerer Pentansäuren nach einem oder mehreren der Ansprüche 1 bis 4, dadurch gekennzeichnet, daß in der Hydroformylierungsstufe der wäßrigen Katalysatorlösung ein Phasentransferreagenz zugesetzt wird.

6. Gemische isomerer Pentansäureester erhalten durch Veresterung von isomeren Pentansäuren nach einem oder mehreren der Patentansprüche 1 bis 6 mit Alkoholen.

7. Gemische isomerer Pentansäureester nach Anspruch 7 dadurch gekennzeichnet, daß die Alkoholkomponente des Estergemisches Pentaerythrit ist.

8. Verwendung von Gemischen isomerer Pentansäureester nach Anspruch 7 oder 8 als Schmiermittel für Kältemittelverdichter, die mit chlorfreien, teilfluorierten Kohlenwasserstoffen als Kältemittel betrieben werden.
